(19) Europäisches Patentamt
European Patent Office
Office européen des brevets

(11) Numéro de publication : **0 282 391 B1**

# (12) FASCICULE DE BREVET EUROPEEN

(45) Date de publication du fascicule du brevet : **21.08.91 Bulletin 91/34**

(51) Int. Cl.[5] : **C07C 69/716,** C07C 67/343

(21) Numéro de dépôt : **88400443.3**

(22) Date de dépôt : **26.02.88**

**(54) Procédé de préparation de composés chimiques trifluorométhyles.**

(30) Priorité : **10.03.87 FR 8703515**

(43) Date de publication de la demande : **14.09.88 Bulletin 88/37**

(45) Mention de la délivrance du brevet : **21.08.91 Bulletin 91/34**

(84) Etats contractants désignés : **AT BE CH DE ES FR GB GR IT LI LU NL SE**

(56) Documents cités :
**US-A- 3 405 163**
**TETRAHEDRON, vol. 20, no. 10, octobre 1964, pages 2163-2166, Pergamon Press Ltd, Oxford, GB; J. BURDON et al.: "The sodium-promoted claisen ester condensations of ethyl perfluoroalkanecarboxylates"**

(73) Titulaire : **RHONE-POULENC CHIMIE**
**25, quai Paul Doumer**
**F-92408 Courbevoie Cédex (FR)**

(72) Inventeur : **Charpentier Morize, Micheline**
**10, allée Diane de Poitiers**
**F-75019 Paris (FR)**
Inventeur : **Aubert, Corinne**
**15, rue Gramme**
**F-75015 Paris (FR)**
Inventeur : **Langlois, Bernard**
**91, rue Duguesclin**
**F-69006 Lyon (FR)**
Inventeur : **Begue, Jean-Pierre**
**241, rue de Charenton**
**F-75012 Paris (FR)**
Inventeur : **Nee, Gérard**
**5, rue des Roses**
**F-94240 L'Hay Les Roses (FR)**

(74) Mandataire : **Le Pennec, Magali et al**
**RHONE-POULENC INTERSERVICES Service Brevets Chimie 25, Quai Paul Doumer**
**F-92408 Courbevoie Cédex (FR)**

EP 0 282 391 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux composés chimiques trifluorométhylés. Elle concerne plus particulièrement de nouveaux dérivés du trifluoroacétoacétate d'éthyle ainsi que leur procédé de préparation.

Il est possible de préparer le trifluoroacétoacétate d'éthyle par exemple par condensation du trifluoroacétate d'éthyle et de l'acétate d'éthyle en présence d'éthanolate de sodium.

Il est connu selon MAC LOUGHLIN (Tetrahedron, (1964), 20, (10), 2163-6) de préparer l'éthyl-2 trifluoroacétoacétate d'éthyle et le méthyl-2 trifluoroacétoacétate d'éthyle par condensation de trifluoroacétate d'éthyle et de butyrate d'éthyle ou de propionate d'éthyle en présence de sodium métallique. Cette condensation est réalisable en laboratoire avec les carboxylates d'alkyle dont la chaîne carboxylate est courte mais la disponibilité de carboxylates insaturés ou cyclaniques est beaucoup plus aléatoire.

Ce type de réaction ne permet pas, notamment, d'obtenir de dérivés allyliques ou des dérivés contenant du soufre fixé sur le carbone situé en position 2 par rapport à la fonction acide, car les acides correspondants ne sont pas disponibles sur le marché.

La présente invention a permis d'atteindre de nouveaux produits par un procédé tout à fait original de synthèse. En effet ce procédé est un procédé d'alkylation du trifluoroacétoacétate d'éthyle.

La présente invention concerne en effet un procédé de préparation de dérivés de formule générale (I) :

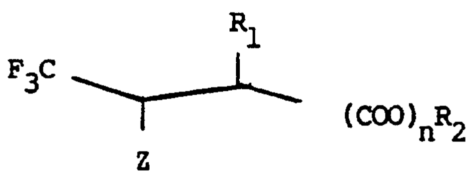

(I)

dans laquelle :

— Z représente un motif bivalent choisi parmi l'oxygène, un groupe dioxolane ou dioxane, un groupe hydrazone,

— $R_1$ représente un motif choisi parmi les groupes nitroaryle, cyanoaryle, alkoxycarbonylaryle, arylthio, trifluorométhylaryle, alkyle, aralkyle, alcènyle, arylalcènyle, aryloxyalkyle, alkylthio, les chaînes alkyles de ces différents groupes peuvent être linéaires, ramifiées, éventuellement substituées et contiennent de préférence de 1 à 12 atomes de carbone ;

— $R_2$ représente un motif choisi parmi les groupes alkyle contenant de 1 à 6 atomes de carbone, hydrogéno, aryle et aralkyle,

— n est un nombre entier égal à 0 ou 1 et quand $n = 0$, $R_2$ est un groupe hydrogéno,

caractérisé en ce que :

* dans une première étape on met en contact :

— un dérivé de formule (II) :

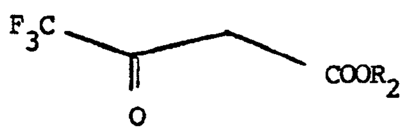

(II)

dans laquelle : $R_2$ a la signification précédente,

— avec un agent de "blocage",

* dans une deuxième étape on met en contact dans un solvant le produit bloqué obtenu à l'étape 1 avec une base et un agent d'alkylation de formule (III) :

$R_1X$) (III)

dans laquelle :

$R_1$ à la signification précédente,

X représente un groupe choisi parmi les radicaux :

F, Cl, Br, I, $R_1SO_4$, $FSO_3$, $CF_3SO_3$,

* dans une (ou deux) étape(s) suivante(s) éventuelle(s) on supprime l'agent de "blocage" et/ou on décarbalcoxyle (formule I, $n = 0$) et/ou on hydrolyse la fonction ester.

Ce procédé s'applique préférentiellement aux dérivés de formule (II) dans lesquels $R_2$ représente un radical éthyle.

L'agent de blocage utilisé dans le procédé de l'invention est un agent évitant que la condensation ne se produise sur l'oxygène du trifluoroacétoacétate d'éthyle et qui donc protège la fonction carbonyle, mais qui doit après alkylation du carbone situé en position 2 pouvoir être éliminé facilement.

Dans le procédé de l'invention on choisit comme agents de blocage :

* les chloroalcanols de formule IV :

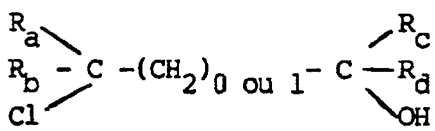

(IV)

dans lesquels $R_a$, $R_b$, $R_c$, $R_c$ identiques ou différents représentent des groupes alkyle ou hydrogéno,

* les époxydes de formule V :

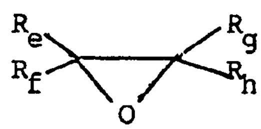

(V)

dans laquelle $R_e$, $R_f$, $R_g$, $R_h$ identiques ou différents représentent des groupes alkyle ou hydrogéno,

* et les hydrazines de formule (VI)

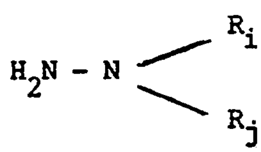

(VI)

dans laquelle $R_i$ et $R_j$ identiques ou différents représentent des groupes alkyle, aralkyle, hydrogéno, l'un au moins d'entre eux n'étant pas hydrogéno.

On préfère tout particulièrement utiliser comme agent de blocage de formule (IV) le chloroéthanol, comme agent de blocage de formule (V) l'oxyde d'éthylène ou de propylène et comme agent de blocage de formule (VI) la diméthyl-1,1 hydrazine.

Parmi les agents d'alkylation de formule (III) on peut citer les iodures de propyle, d'isopropyle, de phényléthyle, de benzyle, de phénylpropyle, de phenylallyle, de butyle, d'isobutyle, d'isopentyle, d'allyle, les bromures de propyle, de phényléthyle, de benzyle, d'isobutyle, d'isopentyle, de phenylallyle, d'allyle, les chlorures de propyle, de benzyle, de nitrophénylthio, d'allyle, les fluorures d'ortho et para nitrophényle, de cyanophényle, d'alcoxycarbonylphényle.

Parmi les bases utilisables dans la seconde étape du procédé selon l'invention on peut choisir le diisopropylamidure de lithium, l'hydrure de potassium ou l'association butyllithium et bis(polyoxaalkyl)amine de formule (VII) :

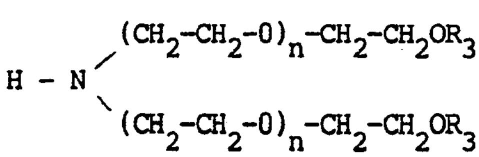

(VII)

dans laquelle n est supérieur ou égal à 1 et inférieur ou égal à 4 et $R_3$ est un radical alkyle contenant 1 à 6 atomes de carbone.

Les solvants utilisables dans le procédé de l'invention peuvent être choisis non limitativement dans la classe des solvants éthérés et de préférence parmi le diéthyléther, le diisopropyléther et le tétrahydrofuranne.

Pour une meilleure mise en oeuvre de l'invention il est avantageux d'ajouter un co-solvant choisi parmi l'hexaméthylphosphoramide, le sulfolane ou la tris(polyoxaalkyl)amine de formule :

$$N — [CHR_4\text{-}CHR_5\text{-}O\text{-}(CHR_6\text{-}CHR_7\text{-}O\text{-}_n)\text{-}R_8]_3 \quad \text{(VIII)}$$

dans laquelle n est supérieur ou égal à 1 et inférieur ou égal à 4 et $R_4$, $R_5$, $R_6$ et $R_7$ identiques ou différents représentent un atome d'hydrogène ou un radical alkyle ayant 1 à 4 atomes de carbone et $R_8$ représente un radical alkyle ayant 1 à 12 atomes de carbone, un radical phényle, alkylphényle, phénylalkyle.

Lorsque l'on désire supprimer l'agent de blocage avant ou après la décarbalcoxylation pour obtenir un composé dans lequel Z représente un atome d'oxygène on utilise avantageusement pour des agents de blocage de formule (IV) ou (V) un trihalogénure d'aluminium ou de bore ; pour des agents de blocage de formule (VI), on utilise avantageusement comme agent de suppression de blocage un système oxydant à base de chrome hexavalent ou un mélange halogénure d'alkyle/éthanol/eau. Parmi ces derniers composés on préfère utiliser le mélange bromure d'éthyle/éthanol/eau.

L'étape de décarbalcoxylation suit habituellement l'étape de suppression de l'agent de blocage (Z représente l'oxygène et n = 0) mais peut ne pas exister (Z représente l'oxygène, un groupe dioxolane, dioxane ou hydrazone et n = 1) ou peut précéder l'étape de suppression de l'agent de blocage (Z ne représente pas l'oxygène et n = 0).

Cette étape de décarbalcoxylation est réalisée :

* dans le cas où Z représente l'oxygène, donc où l'étape de decarbalcoxylation suit l'étape de suppression de l'agent de blocage, par un acide tel que l'acide sulfurique ou par un mélange chlorure de lithium/eau/diméthylformamide,
* dans le cas où Z représente un groupe hydrazone par un mélange chlorure de lithium/eau/diméthylformamide.

Pour provoquer simultanément la décarbalcoxylation et la suppression de l'agent de blocage lorsque Z représente un groupe dioxolane ou dioxane, on utilise avantageusement le complexe trifluorure de bore/acide acétique, le tribromure de bore, le complexe tribromure de bore/diméthylsulfure ou un traitement à la potasse méthanolique suivi d'un traitement à l'acide perchlorique.

L'étape d'hydrolyse précède habituellement l'étape de suppression de l'agent de blocage lorsque Z représente un groupe dioxolane ou dioxane, mais peut ne pas exister. Cette étape d'hydrolyse est réalisée :

* soit en milieu basique (KOH/méthanol),
* soit en milieu acide ($BF_3$/AcOH).

La présente invention concerne aussi de nouveaux produits de formule IX :

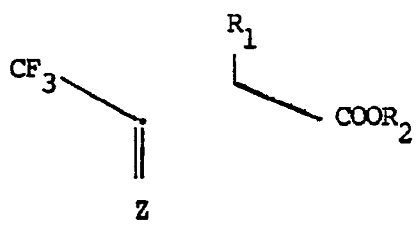

(IX)

dans laquelle :

* Z représente un motif bivalent choisi parmi l'oxygène, un groupe dioxolane ou dioxane, un groupe hydrazone ;
* $R_1$ représente un motif choisi parmi les groupes nitroaryle, cyanoaryle, alkoxycarbonylaryle, trifluorométhylaryle, arylthio, alkyle, aralkyle, alcènyle, arylalcényle, aryloxyalkyle, alkylthio, les chaînes alkyles des ces différents groupes peuvent êtres linéaires, ramifiées, éventuellement substituées et contiennent de préférence de 1 à 12 atomes de carbone ;
* $R_2$ représente un motif choisi parmi les groupes alkyle contenant de 1 à 6 atomes de carbone, hydrogéno, aryle, et aralkyle.

On préfère parmi les composés de formule IX ceux pour lesquels :

Z représente l'oxygène,

$R_2$ représente un groupe éthyle,

$R_1$ représente un groupe nitrophényle ou nitrobenzyle.

Les dérivés de formule (IX) comportant un groupe méthyle à la place du groupe $CF_3$ sont utilisés comme

agent antitumoral ou antimalarique (J. of Medicinal Chemistry, 1971, Vol. 14, n° 11). Les composés de la présente invention sont donc utilisables comme agents pharmacologiquement actifs ou comme intermédiaires en phytothérapie.

Pour une meilleure compréhension de l'invention on va décrire plus en détail le procédé de préparation d'un composé selon l'invention.

Exemple 1 :

Préparation de la N,N diméthylhydrazone dérivée du trifluoroacétoacétate d'éthyle, alkylation par le bromure de benzyle, déblocage et décarbéthoxylation.

A-Préparation de la N,N diméthylhydrazone dérivée du trifluoroacétoacétate d'éthyle (TFAAE) :

Une solution de TFAAE (50 g ; 0,272 mole) et de N,N diméthylhydrazine (19,8 g ; 0,329 mole) dans l'éthanol absolu (80 ml) est portée à reflux pendant 20 h. Après cette période, le mélange réactionnel est concentré sous vide. Le résidu obtenu est distillé et la N,N diméthylhydrazone dérivée du TFAAE est isolée (50,5 g ; 82%).

* $Eb_{17}$ : 85-86°C
* RMN$^1$H (CDCl$_3$/TMS) δ (ppm) : 1,27 (t, 3H, J = 7 Hz, —$CH_2$-$C\underline{H}_3$) ; 2,82 (s, 6H, N($C\underline{H}_3$)$_2$ ; 3,52 (s, 2 H, $C\underline{H}_2$-COOEt) ; 4,20 (q, 2 H, J = 7 Hz, —$C\underline{H}_2$-$CH_3$).
* RMN$^{19}$F ($CDCl_3$/$CFCl_3$) δ (ppm) : – 69,8.
* RMN$^{13}$C (CDCl$_3$/TMS) δ (ppm) : 14,1 (s, $CH_2$-$C\underline{h}_3$) ; 33,3 (s, —$\underline{C}H_2$-COOEt) ; 46,8 (s, N($C\underline{H}_3$)$_2$ ; 61,7 (s, —$\underline{C}H_2CH_3$) ; 121,5 (q, J = 275 Hz, —$\underline{C}F_3$) ; 136 (q, J = 33 Hz, $\underline{C}$=N) ; 168 (s, —$\underline{C}$OOEt).

B-Alkylation de la N,N diméthylhydrazone dérivéee du TFAAE par le bromure de benzyle :

On additionne, à 0°C sous atmosphère d'argon, une solution de diisopropylamidure de lithium (LDA) dans le tétrahydrofurane anhydre (THF) (88 ml ; LDA : 0,097 mole, 1,1 équivalent) [préparée à 0°C à partir de 10 g de diisopropylamine et d'un équivalent de n-Butyllithium (n-BuLi) en solution dans l'hexane] à une solution de N,N-diméthylhydrazone dérivée du TFAAE (19,95 g ; 0,0883 mole) dans le THF (100 ml).

Après 2 heures à 0°C, la température du milieu réactionnel est abaissée à –78°C. La solution est traitée successivement par de l'hexaméthylphosphoramide (HMPT) (31 ml ; 0,178 mole ; 2 équivalents) puis 10 mn après par du bromure de benzyle (16,68 g ; 0,0975 mole ; 1,1 équivalent).

Après 2 heures à –78°C, la température est ramenée lentement à l'ambiante. Après 22 heures, le mélange réactionnel est hydrolysé par l'acide chlorhydrique à 10% et extrait par l'éther. La phase organique est lavée à l'eau (6 fois), séparée, séchée sur du sulfate de magnésium ($MgSO_4$) et évaporée.

Après distillation le produit d'alkylation est obtenu pur (22,3 g ; 80%).

* $Eb_{0,2}$ : 108-110°C
* RMN$^1$H (CDCl$_3$/TMS) δ (ppm) : 1,22 (t, 3H, J = 7 Hz, -O-$CH_2$-$C\underline{H}_3$) ; 2,38 (s, 6 H, N($C\underline{H}_3$)$_2$) ; 4,18 (q, 2 H, J = 7 Hz, -O-$C\underline{H}_2$-$CH_3$) ; 4,78 (m, Δ H, -$C\underline{H}$-) ; 7,23 (m, 5 H arom).
* RMN$^{19}$F ($CDCl_3$/$CFCl_3$) δ (ppm) : - 65,7.
* RMN$^{13}$C (CDCl$_3$/TMS) δ(ppm) : 13,9 (s, -$\underline{C}H_3$) ; 34,4 (s,-$\underline{C}H_2$-) ; 45,7 (s, N($\underline{C}H_3$)$_2$) ; 47,1 (s,-$\underline{C}H$-) ; 61,6 (s, O-$\underline{C}H_2CH_3$) ; 122,4 (q, $\underline{C}F_3$ J = 275 Hz,) ; 126,8 (s, 2 $\underline{C}$ arom.) ; 128,5 (s, 1 $\underline{C}$ arom.) ; 129,1 (s, 2 $\underline{C}$ arom.) ; 138,1 (s, 1 $\underline{C}$ arom.) ; 152,9 (q, J = 31 Hz, -$\underline{C}$ = N-) ; 169,3 (s, -$\underline{C}$OOEt).

C-Déblocage de la N,N diméthylhydrazone dérivée du TFAAE et alkylée par le bromure de benzyle : préparation du ß-céto ester correspondant.

* Par le mélange bromure d'éthyle/éthanol/eau :

A température ambiante, le bromure d'éthyle (51,7 g ; 0,475 mole ; 15 équiv.) est additionné à une solution de produit alkylé (10 g ; 0,0316 mole) dans l'éthanol (100 ml) et l'eau (10 ml). La solution est portée à reflux 72 heures. Après refroidissement, le bromure d'éthyle et l'éthanol en excès sont évaporés sous vide. Le résidu est repris par l'éther et lavé à l'eau (3 fois). La phase organique est séparée, séchée sur sulfate de sodium ($Na_2SO_4$) puis évaporée.

Le produit brut de réaction est chromatographié sur gel de silice (pentane/éther : 95/5). Le ß-cétoester correspondant (7,4 g ; 85%) est obtenu sous forme pure et hydratée.

* RMN $^1H$ ($CDCl_3$/TMS) δ (ppm) : 0,86 (t, 3H, J = 7 Hz, $CH_3$) ; 1,18 (t, 3 H, J = 7 Hz, $CH_3$) ; 3,2 (s, 2 H,, -$CH_2$) 3,33 (t, 2 H, $H_2$) ; 4, 17 (2q superposés, 4 H, 2 - O-$CH_2$) ; 7,17 (m,10 H arom.).

* RMN $^{19}F$ δ (ppm) : - 78,7 (cétone : 74 %). ( $CDCl_3$/$CFCl_3$) : - 84,2 (hydrate : 26 %).

* RMN $^{13}C$ δ (ppm) : 13,6 (s, -$CH_2$-$CH_3$ hyd.) ; 13,8 (s, $CH_2$-$CH_3$-cétone) ; 33,4 (s, $CH_2$-O, hyd.) ; 33,9 (s, - $CH_2$-O, cétone) ; 50,3 (s,- CH hyd.) ; 55,4 (s,-CH, cétone) ; 62,4 (s, O-$CH_2$, hyd.) ; 62,6 (s, O-$CH_2$, cétone) ; 94,1 (q, $C(OH)_2$-, J = 32 Hz, ; 115,5 (q, $CF_3$, J =292 Hz, cétone); 122,6 (q, $CF_3$, J = 292 Hz, hyd.), 127,1 à 137,5 (s, C arom.) 166,9 (s,-COOEt, cétone) ; 174,7 (s, - COOEt, hyd.) ; 186,7 (q, $CF_3$ -C=O ; J = 36 Hz).

* Par l'oxyde chromique dans l'acétone en présence d'acide sulfurique :

La solution aqueuse de réactif de Jones (0,9 ml ; 2 mmoles ; 0,75 équiv.) [préparée à partir de 2,67 g d'oxyde de chrome ($CrO_3$) et de 2,3 ml d'acide sulfurique concentré, diluée par de l'eau jusqu'à un volume de 12 ml] est additionnée à température ambiante à la solution de N,N-diméthylhydrazone alkylée (0,8 g ; 2,53 mmoles) dans l'acétone (15 ml).

Après 3 heures à température ambiante, le milieu réactionnel est hydrolysé et extrait à l'éther. La phase organique est séparée, séchée sur sulfate de magnésium, puis évaporée.

L'analyse par CPG et par RMN$^{19}$ F montre que le produit brut de réaction est composé de :

— 75% de ß-cétoester alkylé et

— 25% de produit de départ.

* Par le chlorochromate de pyridinium :

Le chlorochromate de pyridinium (1,4 g ; 6,5 mmoles ; environ 4 équiv.) est additionné à température ambiante à une solution de N,N-diméthylhydrazone alkylée (0,5 g ; 1,6 mmole) dans le chlorure de méthylène (15 ml). La solution est agité pendant 96 heures à température ambiante. Après cette période, le mélange réactionnel est repris par du pentane et filtré sur gel de silice.

Le produit brut de réaction (0,45 g) est composé uniquement de ß-cétoester alkylé sous forme cétone et hydrate (d'après CPG et RMN$^{19}$ F)

D-Décarbéthoxylation du ß-cétoester alkylé : préparation de la cétone Ø-$(CH_2)_2$-CO-$CF_3$ :

* par le mélange chlorure de lithium/DMF/eau :

Une solution de ß-cétoester alkylé (7 g ; 0,0255 mole) dans le diméthylformamide (DMF) (70 ml) est portée à reflux en présence de chlorure de lithium (LiCl) (2,16 g ; 0,051 mole ; 2 équiv.) et d'eau (0,459 g ; 0,0255 mole) pendant 1h 30. Après refroidissement, le mélange réactionnel est hydrolysé, extrait par l'éther éthylique, lavé à l'eau (5 fois). La phase organique est séparée, séchée sur sulfate de magnésium ($MgSO_4$) puis évaporée.

Le résidu est distillé et la cétone est obtenue pure (4,37 g ; 85%)

| | |
|---|---|
| * $Eb_{20}$ : | 82-84°C |
| * RMN$^1$H | ($CDCl_3$/TMS) δ (ppm) : 3,0 (s, 4 H, $(CH_2)_2$) ; 7,17 (m, 5 H arom.) |
| * RMN$^{19}$F | ($CDCl_3$/$CFCl_3$) δ (ppm) : − 79,8. |
| * RMN$^{13}$C | ($CDCl_3$) δ (ppm) : 28,4 (s, —$CH_2$-O) ; 38 (s, —$CH_2$-$CH_2$) 15,7 (q, $CF_3$, J = 292 Hz) ; 127,7-139,4 (s, C arom.) ; 190,7 (q $CF_3$—C=O ; J = 35 Hz). |

* Par l'acide sulfurique à 40% :

Une solution de ß-céto esteralkylé (3 g ; 0,011 mole) dans l'acide sulfurique à 40% (50 ml) est portée à reflux pendant 20 heures. Après refroidissement, le mélange réactionnel est extrait par de l'éther, lavé à l'eau jusqu'à neutralité. La phase organique est séparée, séchée sur sulfate de magnésium, puis évaporée.

La cétone (1,35 g ; 61%) est obtenue après chromatographie sur gel de silice (pentane/éther : 92/8).

E-Décarbéthoxylation de l'hydrazone dérivée du TFAAE et alkylée par le bromure de benzyle : préparation de la diméthylhydrazone :

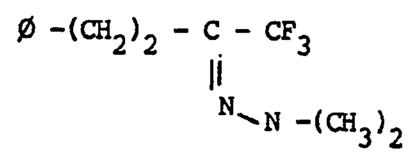

Une solution de N,N-diméthylhydrazone dérivée du TFAAE et alkylée par le bromure de benzyle (0,73 g ; 2,3 mmoles) dans le diméthylformamide (DMF) (10 ml) est portée à reflux en présence de chlorure de lithium (0,2 g ; 4,6 mmoles ; 2 équiv.) et d'eau (0,042 g ; 2,3 mmoles) pendant 24 heures. Après refroidissement, le mélange réactionnel est hydrolysé, extrait par l'éther éthylique, lavé à l'eau (5 fois). La phase organique est séparée, séchée sur sulfate de magnésium puis évaporée.

L'analyse par CPG et par RMN $^{19}$F indique que le produit brut de réaction est composé de :

— 75% de N,N-diméthylhydrazone attendue et,

— 25% de produit de départ.

| | |
|---|---|
| * RMN$^1$H | δ (ppm) (TMS) : 2,4-2,8 (m, 4 H-$(CH_2)_2$—) ; 2,73 (s, 6 H, N-$(CH_3)_2$ ; 7,22 (m, 5 H arom.). |
| * RMN$^{19}$F | ($CDCl_3$/$CFCl_3$) δ (ppm) : − 69,5. |

Example 2 :

A-Préparation du dioxolane dérivé du trifluoroacétoacétate d'éthyle (TFAAE) :

En utilisant la méthode décrite par N.V. KONDRATENKO, E.P. VECHIRKO et L.N. YAGUPOLSKII (Zhur. Org. Khim. 1979, 15, 704.) on forme l'énolate sodique du TFAAE au sein de l'éther éthylique (125 ml), à partir de 50 g (0,27 mole) de TFAAE et de 13,5 g (0,28 mole) d'une suspension d'hydrure de sodium dans l'huile (50%) (l'huile est préalablement éliminée par lavages successifs au pentane). Après 30 mn d'agitation à température ordinaire, l'éther est éliminé sous pression réduite. On ajoute 50 ml de chloroéthanol et on porte à reflux pendant 48 h. Après hydrolyse (glace, puis acide chlorhydrique à 20%), le mélange réactionnel est extrait à l'éther (3 × 100 ml). La phase organique est lavée à neutralité puis séchée sur sulfate de sodium et concentrée. La distillation sous pression réduite permet de séparer 42 g de produit (Rdt : 70%).

* $Eb_{20mm}$ : 95-96°C.
* RMN¹H ($CCl_4$) δ(ppm) : 1,27 (t, 3 H, $CH_3$) ; 2,9 (s, 2H, $CH_2$-$CO_2Et$) ; 4,22 (m, 6 H, $CH_2$).
* RMN¹⁹F ($CCl_4/CFCl_3$) δ (ppm) : – 84.

B-Alkylation du dioxolane dérivé du TFAAE par l'iodure d'octyle en présence de diisopropylamidure de lithium :

On additionne, à –78°C sous atmosphère d'argon, une solution de diisopropylamidure de lithium dans le THF anhydre (47 ml ; LDA : 0,051 mole, 1,1 équivalent) [préparée à 0°C à partir de 5,2 g de diisopropylamine et d'un équivalent de n-butyllithium en solution dans l'hexane] à une solution de dioxolane dérivé du TFAAE (10 g ; 0,046 mole) dans le THF (50 ml).

Après 40 mn à –78°C, la solution est traitée successivement par de l'HMPT (24 ml ; 0,138 mole ; 3 équivalents) puis 10 mn après par de l'iodure d'octyle (12,7 g ; 0,053 mole ; 1,2 équivalent).

La température est ensuite ramenée progressivement à l'ambiante.

Après 4 h à température ambiante, le mélange réactionnel est acidifié par l'acide chlorhydrique à 10% et extrait par l'éther. La phase organique est traitée par une solution d'hydrogénosulfite de sodium, puis lavée à l'eau (4 fois), séparée, séchée sur sulfate de magnésium ($MgSO_4$) et évaporée.

Après distillation, le produit d'alkylation est obtenu pur (13 g ; 82%).

* $Eb_{0,5}$ = 110°C.
* RMN¹H ($CCCl_4$/TMS) δ ppm : – 1,0 (m, 3 H, $(CH_2)_7$-$CH_3$) ; 1,5 (m, 17 H, $(CH_2)_7$ + $CH_2$-$CH_3$) ; 2,9 (m, 1 H; -CH-) ; 4,10 (m, 6 H, O-$CH_2$-$CH_3$ + $CH_2$-$CH_2$ (O O)).
* RMN¹⁹F ($CCl_4/CFCl_3$) δ (ppm) : – 82.

C - Déblocage du dioxolane dérivé du TFAAE et alkylée par l'iodure d'octyle : préparation du ß-céto ester correspondant.

* Par le tribromure de bore :

On ajoute, sous atmosphère d'argon, à une solution de 0,300 g ($0,88.10^{-3}$ mole) de dioxolane dans 10 ml d'hexane refroidie à –10°C, 1,32 ml (1,5 équiv.) d'une solution molaire de $BBr_3$ dans l'hexane. Après 20 mn à –10°C, le mélange réactionnel est hydrolysé et extrait à l'éther (3 fois). Après lavage jusqu'à neutralité, la phase organique est séchée sur sulfate de sodium puis concentrée sous vide. Le produit brut est chromatographié ($SiO_2$, $CH_2Cl_2$). On isole ainsi 225 mg d'octyl-2-trifluoroacétylacétate d'éthyle (Rdt = 86%).

* RMN¹H ($CCCl_4$/TMS) δ ppm : 0,80 (m, 3 H, ($CH_3$) ; 1,60 (m, 14 H, $CH_2$) ; 4,25 (m, 2H, O-$CH_2$—).
* RMN¹⁹F ($CCl_4/CFCl_3$) δ (ppm) : – 77,7 et – 83,3 (forme hydratée).

D-Hydrolyse du dioxolane dérivé du TFAAE (et alkylé par l'iodure d'octyle) suivie d'une décarboxylation par l'acide perchlorique.

Le trifluoroacétyl-2 décanoate d'éthyle éthylène cétal (4 g) est saponifié par traitement avec 60 ml de potasse méthanolique à 10% au reflux pendant 4 jours. Après acidification par l'acide chlorhydrique à 20%, la fraction organique est extraite à l'éther, débarrassée des fractions neutres par traitement acido-basique et séchée sur sulfate de sodium. Après évaporation, on obtient 3,5 g d'acide qui sont directement traités par 15 équivalents (20 ml) d'acide perchlorique à 70%. Après 24 heures de reflux, le mélange réactionnel est repris par l'eau, extrait à l'éther éthylique. La phase éthérée est lavée jusqu'à neutralité et séchée sur sulfate de sodium. On isole ainsi 1,26 g de trifluorométhylnonylcétone (Rdt : 50%).

* RMN¹H ($CCCl_4$/TMS) δ ppm : 0,88 (m, 3 H, $CH_3$) ; 1,60 (m, 14 H, $CH_2$) ; 2,65 (m, 2H, CO-$CH_2$—).
* RMN¹⁹F ($CCl_4/CFCl_3$) δ (ppm) : – 80,33.

E-Déblocage et décarbéthoxylation simultanés du dioxolane dérivé du TFAAE et alkylé par l'iodure d'octyle : préparation de la cétone $CH_3-(CH_2)_8-CO-CF_3$ :

* Par $AcOH/BF_3$ :

On porte à reflux pendant quatre heures 500 mg (1,47 × $10^{-3}$ mole) d'éthylène cétal avec 4,14 g (22 × $10^{-3}$ mole) de complexe $BF_3$, 2 $CH_3COOH$. Après hydrolyse et extraction à l'éther (3 fois), la phase organique est séparée, lavée jusqu'à neutralité puis séchée sur sulfate de sodium. On obtient ainsi 250 mg de trifluorométhylnonylcétone (Rdt = 75%).

* Par le complexe $BBr_3$/sulfure de méthyle :

On ajoute à une solution de 0,200 g (5,9 × $10^{-4}$ mole) d'éthylènecétal du TFAAE alkylé dans 6 ml de dichloroéthane, agitée sous atmosphère d'argon, 0,88 ml (1,5 équiv.) d'une solution 1 M du complexe $BBr_3-(CH_3)_2S$ dans le dichloroéthane (préparée à partir de 0,623 g dans 2 ml). Après 34 heures à reflux, le mélange réactionnel est hydrolysé, extrait à l'éther (3 fois) puis lavé jusqu'à neutralité. La phase organique est séparée puis séchée sur sulfate de sodium et concentrée sous vide. Le produit brut (0,150 g) est constitué de 56% de trifluorométhyl nonylcétone et de 44% d'éthylène cétal de départ, que l'on sépare par chromatographie sur colonne de silice.

F-Alkylation du dioxolane dérivé du TFAAE par l'iodure de propyle, en présence du mélange butyllithium -bis(dioxa-3,6 heptyl)amine (BDA-1) :

On ajoute, sous argon, 1,87 ml d'une solution de butyllithium (2,3 M dans l'hexane) à une solution refroidie à 0°C de 0,945 g (4,3 × $10^{-3}$ mole) de BDAI. Après 15 mn, cette solution d'amidure est additionnée à une solution refroidie à −78°C de 0,750 g (3,3 × $10^{-3}$ mole) de dioxolane dérivé du TFAAE dans 10 ml de THF. Après 15 mn, on ajoute 1 ml (9,9 × $10^{-3}$ mole) d'iodure de n-propyle, puis après 30 mn, on laisse remonter lentement la température à l'ambiante. Après hydrolyse par l'acide chlorhydrique à 10% et extraction à l'éther éthylique (3 fois), la phase organique est lavée avec une solution aqueuse d'hydrogénosulfite de sodium, puis à l'eau avant d'être séchée sur sulfate de sodium. On obtient ainsi 0,650 g d'un produit brut constitué de 50% de composé alkylé, que l'on purifié comme précédemment (distillation sous vide, ou chromatographie).

Exemples 3 à 56 :

Conformément aux exemples 1 et 2, on a réalisé différents exemples d'alkylation avec des agents alkylants $R_1X$ divers, une base, un solvant et un cosolvant différents. Les résultats sont présentés dans les tableaux 1 et 2. Les produits obtenus ont été soumis aux réactions ultérieures de suppression de l'agent de blogage et/ou d'hydrolyse et/ou de décarbalcoxylation. Les résultats sont présentés dans les tableaux 3 à 5.

TABLEAU I

RECAPITULATIF DES ESSAIS D'ALKYLATION AYANT "COMME AGENT DE BLOCAGE" LE DIOXOLANE - LA REACTION EST REALISEE DANS LE TETRAHYDROFURANE

| EX | $R_1$ | X | $R_2$ | BASE | COSOLVANT | Rdt brut RMN/CPG |
|---|---|---|---|---|---|---|
| 3 | $CH_3(CH_2)_2-$ | I | $C_2H_5$ | LDA | HMPT | 95 % |
| 4 | $(CH_3)_2CH-$ | ,, | $C_2H_5$ | LDA | HMPT | 65 % |
| 2-B | $CH_3(CH_2)_7-$ | ,, | $C_2H_5$ | LDA | HMPT | 90 % |
| 5 | $C_6H_5(CH_2)_2-$ | ,, | $C_2H_5$ | LDA | HMPT | 35 % |
| 6 | $CH_3CH_2CH_2-$ | ,, | $C_2H_5$ | LDA | | 27 % |
| 7 | $CH_3CH_2CH_2-$ | ,, | $C_2H_5$ | LDA | TDA | |
| 2-F | $CH_3CH_2CH_2-$ | ,, | $C_2H_5$ | BuLi / BDA | | 59 % |
| 8 | $CH_3(CH_2)_2-$ | ,, | $C_2H_5$ | LDA | Sulfolane | 33 % |
| 9 | $CH_3(CH_2)_2-$ | ,, | $C_2H_5$ | LDA | Sulfolane | 51 % |
| 10 | $CH_3CH_2-$ | $CH_3CH_2SO_4$ | $C_2H_5$ | LDA | HMPT | 90 % |
| 11 | $CH_3(CH_2)_2-$ | Br | $C_2H_5$ | LDA | HMPT | 90 % |
| 12 | $(CH_3)_2CH_2-$ | Br | $C_2H_5$ | LDA | HMPT | 51 % |
| 13 | $C_6H_5(CH_2)_2-$ | ,, | $C_2H_5$ | LDA | HMPT | 35 % |
| 14 | $C_6H_5(CH_2)_3-$ | ,, | $C_2H_5$ | LDA | HMPT | 80 % |
| 15 | $C_6H_5CH_2-$ | ,, | $C_2H_5$ | LDA | HMPT | 90 % |
| 16 | $C_6H_5O(CH_2)_2-$ | ,, | $C_2H_5$ | LDA | HMPT | 56 % |
| 17 | $CH_2{=}CH{-}CH_2-$ | ,, | $C_2H_5$ | LDA | HMPT | 95 % |
| 18 | $CH_2{=}CH{-}CH_2-$ | ,, | $C_2H_5$ | LDA | | 47 % |
| 19 | $C_6H_5CH_2-$ | Cl | $C_2H_5$ | LDA | HMPT | 72 % |
| 20 | $CH_3(CH_2)_2-$ | Cl | $C_2H_5$ | LDA | HMPT | 34 % |
| 21 | $(2\text{-}NO_2\text{-}C_6H_4)S-$ | Cl | $C_2H_5$ | LDA | HMPT | 27 % |
| 22 | $2\text{-}NO_2\text{-}C_6H_4-$ | F | $C_2H_5$ | LDA | HMPT | 44 % |

LDA = Diisopropylamidure de lithium - HMPT = hexaméthylphosphoramide -
*BDA = Bis - (dioxa-3,6 heptyl)amine - TDA = tris-(dioxa-3,6 heptyl)amine -

TABLEAU II

RECAPITULATIF DES ESSAIS D'ALKYLATION AYANT COMME "AGENT DE BLOCAGE" LA DIMETHYLHYDRAZONE

La réaction est réalisée dans le tétrahydrofurane

| EX | $R_1$ | X | $R_2$ | BASE | CO-SOLVANT | RdtBrut% RMN/CPG |
|---|---|---|---|---|---|---|
| 23 | $CH_3(CH_2)_2-$ | I | $C_2H_5$ | LDA | HMPT | 90 % |
| 24 | $CH_2=CH(CH_2)_2-$ | ,, | $C_2H_5$ | LDA | HMPT | 45 % |
| 25 | $C_6H_5(CH_2)_3-$ | ,, | $C_2H_5$ | LDA | HMPT | 80 % |
| 26 | $CH_3(CH_2)_2-$ | ,, | $C_2H_5$ | KH | HMPT | 52 % |
| 27 | $CH_3(CH_2)_2-$ | ,, | $C_2H_5$ | BuLi+BDA | HMPT | 29 % |
| 28 | $2-NO_2-C_6H_4-CH_2-$ | Cl | $C_2H_5$ | LDA | HMPT* | 60 % |
| 29 | $C_6H_5CH_2-$ | Br | $C_2H_5$ | LDA | HMPT | 95 % |
| 30 | $C_6H_5-(CH_2)_2-$ | ,, | $C_2H_5$ | LDA | HMPT | 25 % |
| 31 | $CH_2=CH-CH_2-$ | ,, | $C_2H_5$ | LDA | HMPT | 95 % |
| 32 | $(CH_3)_2C=CH-CH_2-$ | ,, | $C_2H_5$ | LDA | HMPT | 95 % |
| 33 | $C_6H_5CH=CHCH_2$ | ,, | $C_2H_5$ | LDA | HMPT | 80 % |
| 34 | $C_6H_5CH_2-$ | ,, | $C_2H_5$ | LDA | TDA | 80 % |
| 35 | $C_6H_5-CH_2-$ | ,, | $C_2H_5$ | LDA | | 50 % |
| 36 | $(CH_3)_2C=CH-CH_2-$ | ,, | $C_2H_5$ | LDA | | 62 % |
| 1-C | $C_6H_5-CH_2-$ | ,, | $C_2H_5$ | LDA | HMPT | 95 % |
| 37 | $2-NO_2-C_6H_4-$ | F | $C_2H_5$ | LDA | HMPT | 5 % |

LDA = Diisopropylamidure de lithium -
HMPT = hexaméthylphosphoramide - TDA = tris(dioxa-3,6 heptyl)amine
BDA = Bis(trioxa-3,6 heptyl)amine
* 6 équivalents au lieu de 2

Tableau III

SUPPRESSION DE L'AGENT DE BLOCAGE, HYDROLYSE ET DECARBALCOXYLATION DES COMPOSES DE FORMULE I ( Z = dioxolane, n = 1, $R_2 = C_2H_5$)

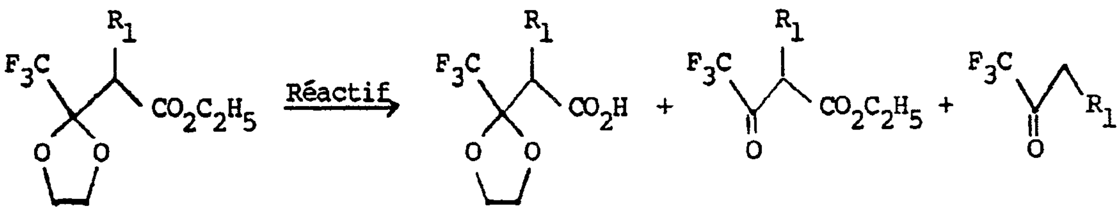


Ia Ib Ic Id

| EX | $R_1$ | Réactif (nombre d'équivalents) | T (°C) | Durée (h) | Rdt analytique (CPG, RMN) % Ia | Ib | Ic | Id |
|---|---|---|---|---|---|---|---|---|
| 38 | $CH_3-(CH_2)_7-$ | $AlI_3$ (2) | 110 | 16 | | | 20 | |
| 2-C | $CH_3-(CH_2)_7-$ | $BBr_3$ (1,5) | -10 | 0,33 | | | 94 | 2 |
| 39 | $CH_3-(CH_2)_7-$ | $BBr_3$ (2,5) | -10 | 0,33 | | | 88 | 6 |
| 40 | $CH_3-(CH_2)_7-$ | $BBr_3$ (5) | -10 | 1 | | | 41 | 56 |
| | | | puis 20 | 2,5 | | | | |
| 41 | $CH_3-(CH_2)_7-$ | $BBr_3$ (5) | -10 | 1 | | | 15 | 72 |
| | | | puis 20 | 7 | | | | |
| 42 | $CH_3-(CH_2)_7-$ | $BCl_3$ (1,5) | 20 | 24 | 40 | | 60 | |
| 43 | $CH_3-(CH_2)_7-$ | $BBr_3,S(CH_3)_2$ | 80 | 17 | 88 | | | 12 |
| 2-E | ,, | ,, | ,, | 34 | 44 | | | 56 |
| 44 | $CH_3-(CH_2)_7-$ | $F_3B$,2AcOH (15) | 80 | 17 | 82 | 15 | | |
| 45 | $CH_3-(CH_2)_7-$ | $F_3B$,2AcOH (15) | 80 | 48 | 56 | 20 | | 11 |
| 46 | $CH_3-(CH_2)_7-$ | $F_3B$,2AcOH (15) | 140 | 2,5 | 4 | 32 | | 58 |
| 2-E | $CH_3-(CH_2)_7-$ | $F_3B$,2AcOH (15) | 140 | 4 | | | | 87 |

--

Tableau III (suite)

SUPPRESSION DE L'AGENT DE BLOCAGE, HYDROLYSE ET DECARBALCOXYLATION DES COMPOSES DE FORMULE I ( Z = dioxolane, n = 1, $R_2$ = $C_2H_5$)

| EX | $R_1$ | Réactif (nombre d'équivalents) | T (°C) | Durée (h) | Rdt analytiques (CPG, RMN) % Ia | Ib | Ic | Id |
|---|---|---|---|---|---|---|---|---|
| 2-D | $CH_3-(CH_2)_7-$ | $KOH/CH_3OH$ | reflux | 96 | | 100 | | |
| 2-D | $CH_3-(CH_2)_7-$ | 1) $KOH/CH_3OH$<br>2) $HClO_4$ à 70 % (15) | reflux<br>reflux | 96<br>24 | | | | 50 |
| 47 | $CH_3-(CH_2)_7-$ | 1) $KOH/CH_3OH$<br>2) $FSO_3H$ (15) | reflux<br>20 | 96<br>89 | | 93 | | 7 |
| 48 | $CH_3-(CH_2)_7-$ | 1)$KOH/CH_3OH$<br>2)$CF_3CO_2H$ (15) | reflux<br>reflux | 96<br>29 | | 98 | | 2 |
| 49 | $C_6H_5-(CH_2)_3-$ | $BBr_3$ (1,5) | -10 | 1 | 20 | | 52 | |
| 50 | $2-NO_2-C_6H_4-$ | $BBr_3$ (1,5) | 0 | 1 | | | 87 | 4 |
| 51 | $2-NO_2-C_6H_4-$ | $BBr_3$ (5) | 20 | 0,25 | | | 70 | 18 |
| 52 | $2-NO_2-C_6H_4-$ | $BBr_3$ (5) | 20 | 1,5 | | | 2 | 80 |

Tableau IV

SUPPRESSION DE L'AGENT DE BLOCAGE DES COMPOSES DE FORMULE :
I ( $Z = N - N(CH_3)_2$ , $n = 1$, $R_2 = C_2H_5$)

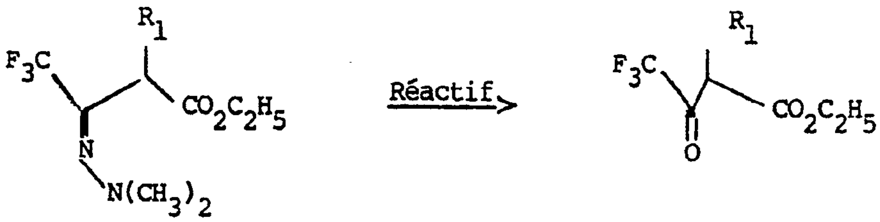


Ie Ic

| EX | $R_1$ | Réactif | T (°C) | Durée | Rdt Ic isolé % |
|---|---|---|---|---|---|
| 1-C | $C_6H_5-CH_2$ | $CrO_3/H_2SO_4/Me_2CO$ | 20 | 3 | |
| 1-C | $C_6H_5-CH_2$ | $C_5H_5NH^{(+)}\ ClCrO_3^{(-)}$ | 20 | 96 | 95 |
| 53 | $C_6H_5-CH_2$ | $CH_3I/C_2H_5OH/H_2O$ | reflux | 48 | 90 |
| 1-C | $C_6H_5-CH_2$ | $C_2H_5Br/C_2H_5OH/H_2O$ | reflux | 72 | 90 |
| 54 | $C_6H_5-(CH_2)_3-$ | $C_2H_5Br/C_2H_5OH/H_2O$ | reflux | 72 | 80 |
| 55 | $(CH_3)_2C=CH-CH_2-$ | $C_2H_5Br/C_2H_5OH/H_2O$ | reflux | 72 | 80 |

Tableau V

DECARBETHOXYLATION DES COMPOSES DE FORMULE
I ( Z = O ou N - N $(CH_3)_2$ , n = 1, $R_2 = C_2H_5$)

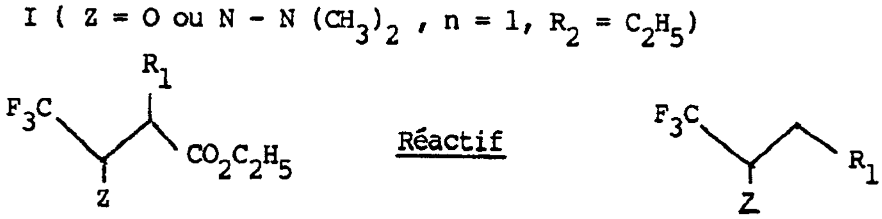


Ic ou Ie — Id (Z = O) ou If [Z = N - N $(CH_3)_2$]

| EX | $R_1$ | Z | Réactif (Equivalents) | T (°C) | Durée (h) | Rdt Id isolé % | Rdt If isolé % |
|---|---|---|---|---|---|---|---|
| 1-D | $C_6H_5-CH_2$ | O | LiCl (2) + $H_2O$ (1) + DMF | reflux | 1,5 | 85 | |
| 1-E | $C_6H_5-CH_2$ | $N-N(CH_3)_2$ | id. | reflux | 24 | | 75 |
| 1-D | $C_6H_5-CH_2$ | O | $H_2SO_4$ à 40 % | reflux | 20 | 80 | |
| 55 | $C_6H_5-(CH_2)_3$ | O | LiCl (2) + $H_2O$ (1) + DMF | reflux | 2 | 90 | |
| 56 | $(CH_3)_2C=CH-CH_2$ | O | id. | reflux | 1,5 | 65 | |

DMF = Diméthylformamide

## Revendications

1. Procédé de préparation de dérivés de formule générale (I) :

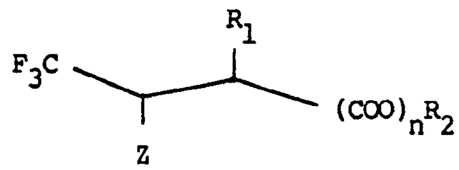 (I)

dans laquelle :

— Z représente un motif bivalent choisi parmi l'oxygène, un groupe dioxolane ou dioxane, un groupe hydrazone,

— $R_1$ représente un motif choisi parmi les groupes nitroaryle, cyanoaryle, alkoxycarbonylaryle, arylthio, trifluorométhylaryle, alkyle, aralkyle, alcényle, arylalcényle, aryloxyalkyle, alkylthio, les chaînes alkyles de ces différents groupes peuvent êtres linéaires, ramifiées, éventuellement substituées et contiennent de préférence de 1 à 12 atomes de carbone ;

— $R_2$ représente un motif choisi parmi les groupes alkyle contenant de 1 à 6 atomes de carbone, hydrogéno, aryle, et aralkyle,

— n est un nombre entier égal à 0 ou 1 et quand n = 0, $R_2$ est un groupe hydrogéno, caractérisé en ce que :

* dans une première étape on met en contact :

— un dérivé de formule (II) :

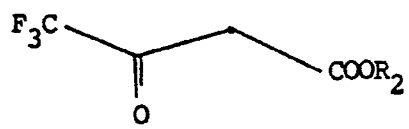 (II)

dans laquelle : $R_2$ a la signification précédente,

— avec un agent de "blocage",

* dans une deuxième étape on met en contact dans un solvant le produit bloqué obtenu à l'étape 1 avec une base et un agent d'alkylation de formule (III) :

$$R_1X \quad (III)$$

dans laquelle $R_1$ à la signification précédente,

X représente un groupe choisi parmi les radicaux : F, Cl, Br, I, $R_1SO_4$, $FSO_3$, $CF_3SO_3$,

* dans une (ou deux) étape(s) suivante(s) éventuelle(s) on supprime l'agent de "blocage" et/ou on décarbalcoxyle et/ou on hydrolyse la fonction ester.

2. Procédé selon la revendication 1, caractérisé en ce que dans la formule (II) le groupe $R_2$ représente un radical éthyle.

3. Procédé selon la revendication 1, caractérisé en ce que l'agent "bloquant" est choisi parmi les chloroalcanols de formule (IV) :

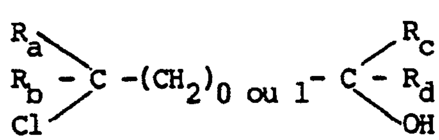 (IV)

dans lesquels $R_a$, $R_b$, $R_c$, $R_c$ identiques ou différents représentent des groupes alkyle ou hydrogéno,

* les époxydes de formule (V) :

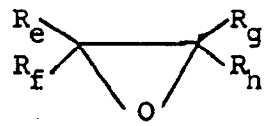 (V)

dans laquelle $R_e$, $R_f$, $R_g$, $R_h$ identiques ou différents représentent des groupes alkyle ou hydrogèno,

* et les hydrazines de formule (VI) :

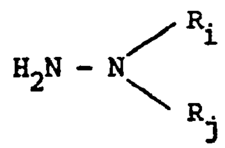 (VI)

dans laquelle $R_i$ et $R_j$ identiques ou différents représentent des groupes alkyle, aralkyle, hydrogéno, l'un au moins d'entre eux n'étant pas hydrogéno.

4. Procédé selon la revendication 1, caractérisé en ce que la base est choisie parmi le diisopropylamidure de lithium, l'hydrure de potassium et l'association butyllithium et bis(polyoxaalkyl)amine de formule (VII) :

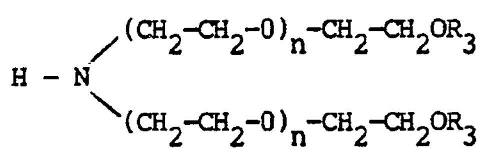 (VII)

dans laquelle n est supérieur ou égal à 1 et inférieur ou égal à 4 et $R_3$ est un radical alkyle contenant 1 à 6 atomes de carbone.

5. Procédé selon la revendication 1, caractérisé en ce que le solvant est choisi parmi le diéthyléther, le diisopropyléther et le tétrahydrofurane.

6. Procédé selon les revendications 1 et 5, caractérisé en ce que l'on ajoute un co-solvant choisi parmi l'hexaméthylphosphoramide, le sulfolane ou la tris(polyoxaalkyl)amine de formule VIII :

$$N\text{-}[CHR_4\text{-}CHR_5\text{-}O\text{-}(CHR_6\text{-}CHR_7\text{-}O\text{-})_n\text{-}R_8]_3 \quad \text{(VIII)}$$

dans laquelle n est supérieur ou égal à 1 et inférieur ou égal à 4 et $R_4$, $R_5$, $R_6$ et $R_7$ identiques ou différents représentent un atome d'hydrogène ou un radical alkyle ayant 1 à 4 atomes de carbone et $R_8$ représente un radical alkyle ayant 1 à 12 atomes de carbone, un radical phényle, alkylphényle, phénylalkyle.

7. Procédé selon la revendication 1, caractérisé en ce que dans l'étape de suppression de l'agent de blocage de formule IV (ou V), on utilise un trihalogénure d'aluminium ou de bore.

8. Procédé selon la revendication 1, caractérisé en ce que dans l'étape de suppression de l'agent de blocage de formule (VI), on utilise un système oxydant à base de chrome hexavalent ou un mélange halogénure d'alkyle/éthanol/eau.

9. Procédé selon la revendication 8, caractérisé en ce que le suppresseur de l'agent de blocage de formule (VI) est le réactif suivant :

bromure d'éthyle/éthanol/eau.

10. Procédé selon la revendication 1, caractérisé en ce que dans l'étape de décarbalcoxylation :

* lorsque Z représente l'oxygène, on utilise un acide tel que $H_2SO_4$ ou un mélange chlorure de lithium, eau et diméthylformamide,

* lorsque Z représente un groupe hydrazone, on utilise un mélange chlorure de lithium, eau et diméthylformamide.

11. Procédé selon la revendication 1, caractérisé en ce que l'on réalise simultanément l'étape de suppression de l'agent de blocage et l'étape de décarbalcoxylation en utilisant un des réactifs choisis parmi l'acide perchlorique, le complexe trifluorure de bore, acide acétique, le tribromure de bore ou le complexe tribromure de bore/diméthylsulfure.

## Patentansprüche

1. Verfahren zur Herstellung von Verbindungen der allgemeinen Formel (I)

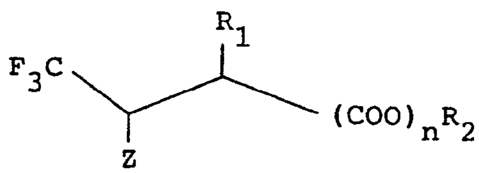

(I)

in der

— Z einen aus Sauerstoff, einer Dioxolan- oder Dioxan- und einer Hydrazongruppe ausgewählten zweiwertigen Rest bedeutet,

— $R_1$ einen Rest bedeutet, der aus den Gruppen Nitroaryl, Cyanoaryl, Alkoxycarbonylaryl, Arylthio, Trifluormethylaryl, Alkyl, Aralkyl, Alkenyl, Arylalkenyl, Aryloxyalkyl und Alkylthio ausgewählt ist, wobei die Alkylketten dieser verschiedenen Gruppen gegebenenfalls substituiert, geradkettig und verzweigt sein können und vorzugsweise 1-12 Kohlenstoffatome enthalten,

— $R_2$ einen aus Alkylgruppen mit 1-6 Kohlenstoffatomen, Wasserstoff, Aryl- und Aralkylgruppen ausgewählten Rest bedeutet und

— n gleich 0 oder 1, und wenn n = 0, $R_2$ Wasserstoff ist, dadurch gekennzeichnet, daß man

* in einer ersten Stufe

— eine Verbindung der Formel (II)

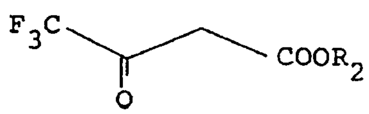

(II)

in der $R_2$ obige Bedeutung hat,

— mit einem Blockierungsmittel umsetzt,

* in einer zweiten Stufe das in Stufe 1 erhaltene blockierte Produkt in einem Lösungsmittel mit einer Base und einem Alkylierungsmittel der Formel (III)

$$R_1X \quad (III)$$

in der $R_1$ obige Bedeutung hat und X eine aus den Resten F, Cl, Br, I, $R_1\ SO_4$, $FSO_3$ und $CF_3\ SO_3$ ausgewählte Gruppe bedeutet, in Berührung bringt und

* in einer (oder zwei) gegebenenfalls folgenden Stufe(n) das Blockierungsmittel entfernt und/oder die Esterfunktion decarbalkoxyliert und/oder hydrolysiert.

2. Verfahren nach Anspruch 1, dadurch gekennzeichnet, daß in der Formel (II) die Gruppe $R_2$ einen Ethylrest bedeutet.

3. Verfahren nach Anspruch 1, dadurch gekennzeichnet, daß das Blockierungsmittel aus den Chloralkanolen der Formel (IV)

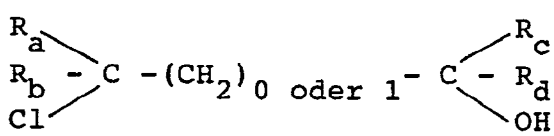

(IV)

in der $R_a$, $R_b$, $R_c$ und $R_c$ gleich oder verschieden sind und Alkylgruppen oder Wasserstoff bedeuten,

* Epoxiden der Formel (V)

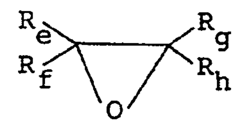 (V)

in der $R_e$, $R_f$, $R_g$ und $R_h$ gleich oder verschieden sind und Alkylgruppen oder Wasserstoff bedeuten, und

* Hydrazinen der Formel (VI)

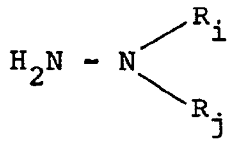 (VI)

in der $R_i$ und $R_j$ gleich oder verschieden sind und Alkyl- und Aralkylgruppen und Wasserstoff bedeuten, wobei wenigstens eine davon kein Wasserstoff ist, ausgewählt ist.

4. Verfahren nach Anspruch 1, dadurch gekennzeichnet, daß die Base aus Lithiumdiisopropylamid, Kaliumhydrid und dem Umsetzungsprodukt aus Butyllithium und Bis(polyoxaalkyl)amin der Formel (VII)

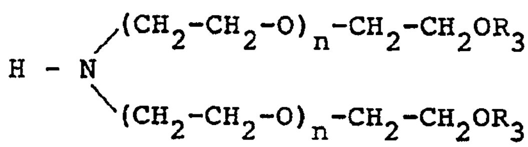 (VII)

in der n gleich 1 bis 4 und $R_3$ ein 1 bis 6 Kohlenstoffatome enthaltender Alkylrest ist, ausgewählt ist.

5. Verfahren nach Anspruch 1, dadurch gekennzeichnet, daß das Lösungsmittel aus Diethylether, Diisopropylether und Tetrahydrofuran ausgewählt ist.

6. Verfahren nach den Ansprüchen 1 und 5, dadurch gekennzeichnet, daß man ein Co-Lösungsmittel zugibt, das aus Hexamethylphosphoramid, Sulfolan oder Tris(polyoxaalkyl)amin der Formel VIII

$$N\text{-}[CHR_4\text{-}CHR_5\text{-}O\text{-}(CHR_6\text{-}CHR_7\text{-}O\text{-})_n\text{-}R_8]_3 \quad \text{(VIII)}$$

ausgewählt ist, in der n gleich 1 bis 4 ist, $R_4$, $R_5$, $R_6$ und $R_7$ gleich oder verschieden sind und ein Wasserstoffatom oder einen 1 bis 4 Kohlenstoffatome enthaltenden Alkylrest bedeuten und $R_8$ einen 1 bis 12 Kohlenstoffatome enthaltenden Alkylrest, einen Phenyl-, Alkylphenyl- und Phenylalkylrest bedeutet.

7. Verfahren nach Anspruch 1, dadurch gekennzeichnet, daß man in der Stufe der Entfernung des Blockierungsmittels der Formel IV (oder V) ein Aluminium- oder Bortrihalogenid verwendet.

8. Verfahren nach Anspruch 1, dadurch gekennzeichnet, daß man in der Stufe der Entfernung des Blockierungsmittels der Formel (VI) ein Oxidationssystem auf der Grundlage des sechswertigen Chroms oder ein Gemisch aus Alkylhalogenid, Ethanol und Wasser verwendet.

9. Verfahren nach Anspruch 8, dadurch gekennzeichnet, daß das Mittel zur Entfernung des Blockierungsmittels der Formel (VI) das Reagens aus Ethylbromid, Ethanol und Wasser ist.

10. Verfahren nach Anspruch 1, dadurch gekennzeichnet, daß man in der Decarbalkoxylierungsstufe

* eine solche Säure wie $H_2SO_4$ oder ein Gemisch aus Lithiumchlorid, Wasser und Dimethylformamid, wenn Z Sauerstoff, und

* ein Gemisch aus Lithiumchlorid, Wasser und Dimethylformamid verwendet, wenn Z eine Hydrazongruppe bedeutet.

11. Verfahren nach Anspruch 1, dadurch gekennzeichnet, daß man gleichzeitig die Stufe der Entfernung des Blockierungsmittels und die der Decarbalkoxylierung unter Verwendung eines der Reagenzien durchführt, die aus Perchlorsäure, dem Komplex aus Bortrifluorid und Essigsäure, Bortribromid oder dem Komplex aus Bortribromid und Dimethylsulfid ausgewählt sind.

## Claims

1. Process for the preparation of derivatives of the general formula (I) :

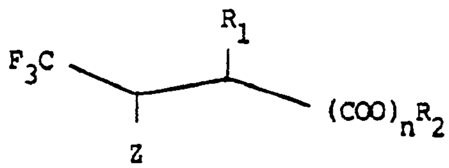

(I)

in which :

Z represents a divalent unit chosen from among oxygen, a dioxolane or dioxane group or a hydrazone group,

$R_1$ represents a unit chosen from among the nitroaryl, cyanoaryl, alkoxycarbonylaryl, arylthio, trifluoromethylaryl, alkyl, aralkyl, alkenyl, arylalkenyl, aryloxyalkyl and alkylthio groups, it being possible for the alkyl chains of these various groups to be linear, branched and optionally substituted, the chains preferably containing from 1 to 12 carbon atoms,

$R_2$ represents a unit chosen from amoung alkyl groups containing 1 to 6 carbon atoms, hydrogen, aryl and aralkyl groups,

n is an integer equal to 0 or 1 and if n is 0, $R_2$ is a hydrogen group, characterised in that

in a first stage a derivative of the formula (II) :

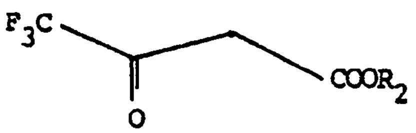

(II)

in which $R_2$ has the above meaning, is brought into contact with a "blocking" agent,

in a second stage, the blocked product obtained in stage 1 is brought into contact, in a solvent, with a base and an alkylating agent of the formula (III) :

$$R_1X \quad (III)$$

in which :

$R_1$ has the above meaning and

X represents a group chosen from among the radicals F, Cl, Br, I, $R_1SO_4$, $FSO_3$ and $CF_3SO_3$, and

in one (or two) subsequent optional stage(s) the "blocking" agent is eliminated and/or the ester group is decarbalkoxylated and/or hydrolysed.

2. Process according to claim 1, characterised in that in the formula (II) the group $R_2$ represents an ethyl radical.

3. Process according to claim 1, characterised in that the "blocking" agent is chosen from among the chloroalkanols of the formula (IV) :

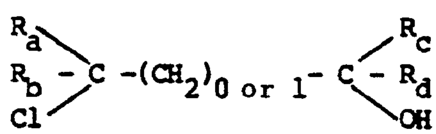

(IV)

in which $R_a$, $R_b$, $R_c$ and $R_d$, which may be identical or different, represent alkyl groups or hydrogen,

the epoxides of the formula (V) :

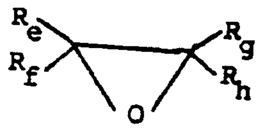

(V)

in which $R_e$, $R_f$, $R_g$ and $R_h$, which may be identical or different, represent alkyl groups or hydrogen,

and the hydrazines of the formula (VI) :

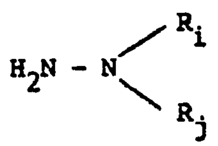

(VI)

in which $R_i$ and $R_j$, which may be identical or different represent alkyl or aralkyl groups or hydrogen, at least one of $R_i$ and $R_j$ not being hydrogen.

4. Process according to claim 1, characterised in that the base is chosen from among lithium diisopropylamide, potassium hydride and the combination of butyllithium and bis(polyoxaalkyl)amine of the formula (VII) :

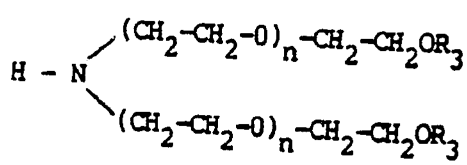

(VII)

in which n is greater than or equal to 1 and less than or equal to 4 and $R_3$ is an alkyl radical containing 1 to 6 carbon atoms.

5. Process according to claim 1, characterised in that the solvent is chosen from among diethyl ether, diisopropyl ether and tetrahydrofuran.

6. Process according to claims 1 and 5, characterised in that a cosolvent is added which is chosen from among hexamethylphosphoramide, sulpholane or the tris-(polyoxaalkyl)amine of the formula VIII :

$$N\text{-}[CHR_4\text{-}CHR_5\text{-}O\text{-}(CHR_6\text{-}CHR_7\text{-}O\text{-})_n\text{-}R_8]_3 \quad (VIII)$$

in which n is greater than or equal to 1 and less than or equal to 4 and $R_4$, $R_5$, $R_6$ and $R_7$, which may be identical or different, represent a hydrogen atom or an alkyl radical having 1 to 4 carbon atoms, and $R_8$ represents an alkyl radical having 1 to 12 carbon atoms or a phenyl, alkylphenyl or phenylalkyl radical.

7. Process according to claim 1, characterised in that an aluminium trihalide or boron trihalide is used in the stage of eliminating the blocking agent of the formula IV (or V).

8. Process according to claim 1, characterised in that an oxidising system based on hexavalent chromium or a mixture of alkyl halide/ethanol/water is used in the stage of eliminating the blocking agent of the formula (VI).

9. Process according to claim 8, characterised in that the reagent used to eliminate the blocking agent of the formula (VI) is ethyl bromide/ethanol/water.

10. Process according to claim 1, characterised in that in the decarbalkoxylation stage :

if Z represents oxygen, an acid such as $H_2SO_4$, or a mixture of lithium chloride, water and direthylformamide is used, and

if Z represents a hydrazone group, a mixture of lithium chloride, water and direthylformamide is used.

11. Process according to claim 1, characterised in that the stage of eliminating the blocking agent and the stage of decarbalkoxylation are carried out simultaneously, using a reagent chosen from among perchloric acid, the boron trifluoride/acetic acid complex, boron tribromide or the boron tribromide/dimethylsulphide complex.